Europäisches Patentamt
European Patent Office
Office européen des brevets

(11) Veröffentlichungsnummer: **0 182 188**
**A2**

# (12) EUROPÄISCHE PATENTANMELDUNG

(21) Anmeldenummer: **85114020.2**

(22) Anmeldetag: **05.11.85**

(51) Int. Cl.4: **C 07 D 249/08**, C 07 D 409/06, A 01 N 43/653 // C07D303/22, C07C49/175, C07C49/167

(30) Priorität: **14.11.84 DE 3441526**

(43) Veröffentlichungstag der Anmeldung: **28.05.86 Patentblatt 86/22**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Büchel, Karl Heinz, Prof. Dr., Dabringhausener Strasse 42, D-5093 Burscheid (DE)**
Erfinder: **Kraatz, Udo, Dr., Andreasstrasse 22a, D-5090 Leverkusen 1 (DE)**
Erfinder: **Reinecke, Paul, Dr., Steinstrasse 8, D-5090 Leverkusen 3 (DE)**

(54) **Triazolylmethylcarbinol-arylacetale.**

(57) Die Erfindung betrifft neue Triazolylmethylcarbinol-arylacetale, ein Verfahren zu ihrer Herstellung und ihre Verwendung als Fungizide.

Die Verbindungen der allgemeinen Formel

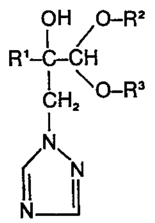

(I)

in welcher

$R^1$, $R^2$ und $R^3$ die in der Beschreibung angegebene Bedeutung besitzen,

werden erhalten, wenn man die entsprechenden Oxirane mit 1,2,4-Triazol in Gegenwart eines Verdünnungsmittels und gegebenenfalls in Gegenwart einer Base umsetzt, und gegebenenfalls noch anschließend eine Säure oder ein Metallsalz addiert.

Die neuen Verbindungen sind gegen phytopathogene Pilze wirksam und können als Pflanzenschutzmittel, insbesondere in Getreide- und Reiskulturen, Verwendung finden.

EP 0 182 188 A2

BAYER AKTIENGESELLSCHAFT
Konzernverwaltung RP
Patentabteilung

5090 Leverkusen, Bayerwerk
Slr/Bas/bo/c
Ia

# Triazolylmethylcarbinol-arylacetale

Die Erfindung betrifft neue Triazolylmethylcarbinol-arylacetale, ein Verfahren zu ihrer Herstellung, sowie ihre Verwendung als Fungizide.

Es ist bereits bekannt, daß bestimmte, gegebenenfalls im Phenylteil substituierte 3,3-Dimethyl-1-phenoxy-2-(1,2,4-triazol-1-yl-methyl)-2-butanole gute fungizide Eigenschaften aufweisen (vgl. DE-OS 31 06 076 und DE-OS 32 02 601). Die Wirkung dieser Verbindungen ist jedoch, insbesondere bei niedrigen Aufwandmengen und -konzentrationen, nicht immer voll befriedigend.

Es wurden neue Triazolylmethylcarbinol-arylacetale der allgemeinen Formel (I)

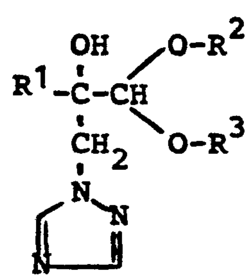

(I)

in welcher

$R^1$ für jeweils gegebenenfalls substituiertes Alkyl, Alkenyl, Cycloalkyl, Heterocyclyl oder Aryl steht und

$R^2$ und $R^3$ unabhängig voneinander für jeweils gegebenenfalls substituiertes Aryl oder Heterocyclyl stehen,

und deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen. Sind die Reste $R^2$ und $R^3$ in der allgemeinen Formel (I) nicht identisch, erhält man ein weiteres asymmetrisches Kohlenstoffatom und somit diastereomere Gemische von (I).

Weiterhin wurde gefunden, daß man die Triazolylmethylcarbinol-arylacetale der Formel (I)

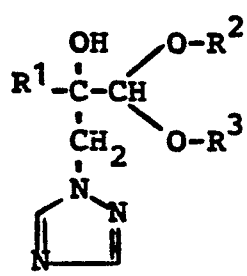

(I)

in welcher

$R^1$ für jeweils gegebenenfalls substituiertes Alkyl, Alkenyl, Cycloalkyl, Heterocyclyl oder Aryl steht, und

$R^2$ und $R^3$ unabhängig voneinander für jeweils gegebenenfalls substituiertes Aryl oder Heterocyclyl stehen,

erhält, wenn man Oxirane der Formel (II)

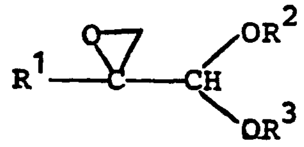

(II)

in welcher

$R^1$, $R^2$ und $R^3$ die oben angegebene Bedeutung haben,

mit 1,2,4-Triazol der Formel (III)

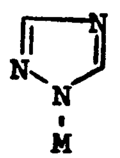

(III)

in welcher

M für Wasserstoff oder ein Alkalimetalläquivalent steht,

in Gegenwart eines Verdünnungsmittels und gegebenenfalls in Gegenwart einer Base umsetzt, und gegebenenfalls noch anschließend eine Säure oder ein Metallsalz addiert.

Die neuen Triazolylmethylcarbinol-arylacetale der allgemeinen Formel (I) sowie deren pflanzenverträglichen

Säureadditionssalze und Metallsalzkomplexe weisen starke fungizide Eigenschaften auf.

Überraschenderweise zeigen die erfindungsgemäßen Triazolylmethylcarbinol-arylacetale der Formel (I) bessere fungizide Wirkungen als aus dem Stand der Technik bekannte 3,3-Dimethyl-1-phenoxy-2-(1,2,4-triazol-1-yl-methyl)-2-butanole, welche konstitutionell und wirkungsmäßig naheliegende Verbindungen sind. Die erfindungsgemäßen Wirkstoffe stellen somit eine Bereicherung der Technik dar.

Die erfindungsgemäßen Verbindungen sind durch die Formel (I) allgemein definiert. In dieser Formel stehen vorzugsweise

$R^1$ für geradkettiges oder verzweigtes Alkyl mit 1 bis 4 Kohlenstoffatomen, für gegebenenfalls einfach oder mehrfach, gleich oder verschieden substituiertes Cycloalkyl mit 3 bis 7 Kohlenstoffatomen, wobei als Substituenten genannt seien: Halogen, Alkyl mit 1 bis 4 Kohlenstoffatomen und Alkoxy mit 1 oder 2 Kohlenstoffatomen, ferner für jeweils gegebenenfalls einfach oder mehrfach, gleich oder verschieden substituiertes Phenyl bzw. fünfgliedriges Heterocyclyl mit einem Sauerstoff- oder Schwefelatom oder ein oder zwei Stickstoffatomen, wobei als Substituenten die unten bei $R^2$ genannten Phenylsubstituenten in Frage kommen, sowie für die Gruppierungen

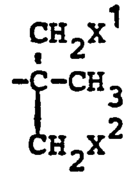 und 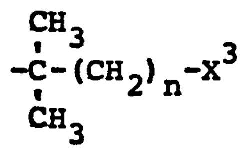 ,

wobei

$X^1$ für Halogen steht,

$X^2$ für Wasserstoff oder Halogen steht,

$X^3$ für Alkyl, Alkoxy und Alkylthio mit jeweils 1 bis 4 Kohlenstoffatomen, für Halogenalkoxy und Halogenalkylthio mit jeweils 1 oder 2 Kohlenstoffatomen und 1 bis 5 gleichen oder verschiedenen Halogenatomen, wie Fluor- und Chloratomen, ferner für Alkenyl mit 2 bis 6 Kohlenstoffatomen, für Alkoxycarbonyl mit 1 bis 4 Kohlenstoffatomen im Alkylteil, für Cyano sowie für gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten die unten bei $R^2$ genannten Phenylsubstituenten in Frage kommen, und

n für die Zahlen 0, 1 oder 2 steht,

$R^2$ und $R^3$ in Formel (I) stehen für Phenyl oder für sechsgliedriges Heterocyclyl mit einem oder zwei

Stickstoffatomen, wobei diese Reste jeweils einfach oder mehrfach substituiert sein können durch Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, durch Cyclohexyl, Dialkylamino mit 1 bis 4 Kohlenstoffatomen in jedem Alkylteil, durch Nitro, Cyano, Alkoxycarbonyl mit 1 bis 4 Kohlenstoffatomen im Alkylteil, sowie gegebenenfalls durch Halogen substituiertes Phenyl.

Besonders bevorzugt sind diejenigen Verbindungen der Formel (I), in denen

$R^1$ für tert.-Butyl oder Isopropyl steht, für jeweils gegebenenfalls einfach bis dreifach, gleich oder verschieden substituiertes Cyclopropyl, Cyclopentyl oder Cyclohexyl steht, wobei als Substituenten genannt seien: Methyl, Ethyl, Isopropyl, Methoxy und Ethoxy, ferner für gegebenenfalls einfach bis dreifach, gleich oder verschieden substituiertes Phenyl steht, wobei als Substituenten genannt seien: Fluor, Chlor, Methyl, Trifluormethyl, Phenyl und Chlorphenyl, sowie für Thienyl steht, welches gegebenenfalls einfach bis dreifach durch Fluor, Chlor, Brom und Methyl substituiert sein kann, sowie für die Gruppierungen

$$\begin{array}{c} CH_2-X^1 \\ | \\ -C-CH_3 \\ | \\ CH_2-X^2 \end{array} \quad \text{und} \quad \begin{array}{c} CH_3 \\ | \\ -C-(CH_2)_n-X^3 \\ | \\ CH_3 \end{array}$$

steht, wobei

$X^1$ für Fluor oder Chlor steht,

$X^2$ für Wasserstoff, Fluor oder Chlor steht,

$X^3$ für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für jeweils gegebenenfalls substituiertes Phenyl, Phenoxy, Phenylthio, Phenylmethoxy oder Phenylmethylthio steht, wobei jeweils als Phenylsubstituenten genannt seien: Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl und

n für die Zahlen 0, 1 oder 2 steht, und

$R^2$ und $R^3$ für Phenyl und für 2- oder 3-Pyridyl steht, wobei die genannten Reste durch Fluor, Chlor, Methyl, Methoxy, Trifluormethyl, Phenyl oder Chlorphenyl substituiert sein können.

Bevorzugte erfindungsgemäße Verbindungen sind auch Additionsprodukte aus Säuren und denjenigen Triazolylmethylcarbinol-arylacetalen der Formel (I), in denen

die Substituenten $R^1$, $R^2$, $R^3$ die Bedeutung haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Verbindungen der Formel (I), in denen die Substituenten $R^1$, $R^2$ und $R^3$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man zur Herstellung der erfindungsgemäßen Verbindungen der allgemeinen Formel (I) beispielsweise 2-(1-Fluor-2-methyl-prop-2-yl)-2-/(4-chlorphenoxy)-(4-chlor-2-methylphenoxy)-methyl/-oxiran und 1,2,4-Triazol als Ausgangsstoffe, so kann der Reaktionsablauf durch das folgende Formelschema wiedergegeben werden:

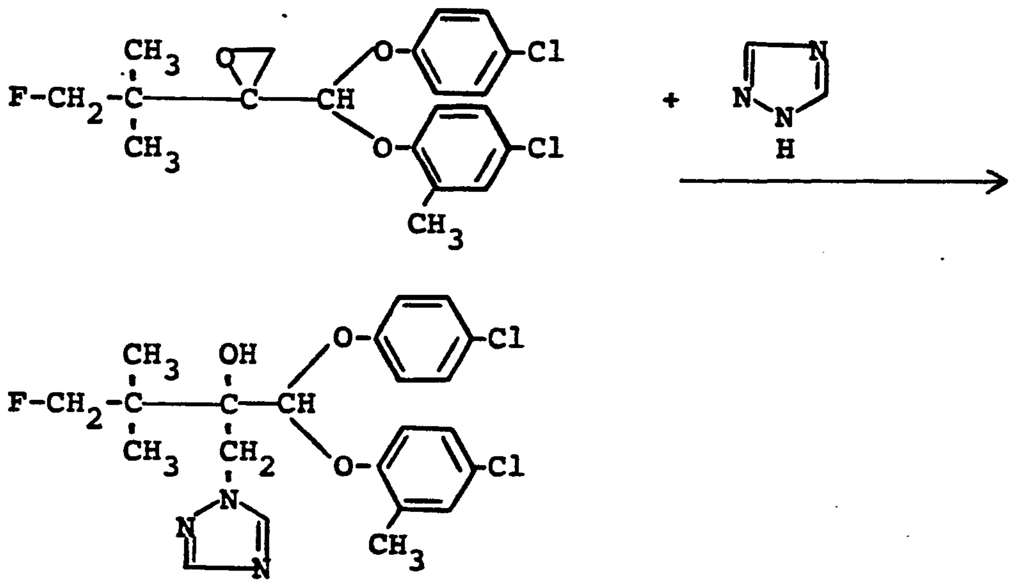

Die bei der Durchführung des erfindungsgemäßen Verfahrens als Ausgangsstoffe zu verwendenden Oxirane sind durch die Formel (II) allgemein definiert. In dieser

Formel haben $R^1$, $R^2$ und $R^3$ vorzugsweise die Bedeutungen, die bereits im Zusammenhang mit der Beschreibung der erfindungsgemäßen Stoffe der Formel (I) vorzugsweise für diese Substituenten genannt wurden.

Die Oxirane der Formel (II) sind noch nicht bekannt. Sie stellen interessante Zwischenprodukte dar und können in allgemein bekannter Art und Weise erhalten werden, indem man Ketone der Formel (IV)

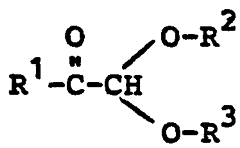

(IV)

in welcher

$R^1$, $R^2$, $R^3$ die oben angegebene Bedeutung haben,

entweder

α) mit Dimethyloxosulfonium-methylid der Formel (V)

$$(CH_3)_2 \overset{\delta+}{S}O \overset{\delta-}{C}H_2 \qquad (V)$$

in Gegenwart eines Verdünnungsmittels umsetzt,

oder

ß) mit Trimethylsulfonium-methylsulfat der Formel (VI)

$$\left[(CH_3)_2S^{(+)}\right] \quad (CH_3SO_4)^{(-)} \qquad (VI)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (IV) lassen sich nach im Prinzip bekannten Verfahren herstellen (vgl. z.B. die Herstellungsbeispiele).

Das bei der Verfahrensvariante (α) benötigte Dimethyloxosulfoniummethylid der Formel (V) ist bekannt (vgl. J. Amer. Chem. Soc. 87, 1363 - 1364 (1965)). Es wird bei der obigem Umsetzung in frisch zubereitetem Zustand verarbeitet, indem man es in situ durch Umsetzung von Trimethyloxosulfoniumiodid mit Natriumhydrid, Natriumamid bzw. Kalium-tert.-butylat in Gegenwart eines Verdünnungsmittels erzeugt.

Das bei der Verfahrensvariante (β) benötigte Trimethylsulfonium-methylsulfat der Formel (VI) ist ebenfalls bekannt (vgl. Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (α) des Verfahrens zur Herstellung der Oxirane der Formel (II) kommt als Verdünnungsmittel vorzugsweise Dimethylsulfoxid in Frage.

Die Reaktionstemperaturen können bei der oben beschriebenen Verfahrensvariante (α) innerhalb eines größeren Bereiches variiert werden. Im allgemeinen arbeitet man bei Temperaturen zwischen 20°C und 80°C.

Die Durchführung des Verfahrens zur Herstellung der Oxirane der Formel (II) nach der Variante (α) und die Aufarbeitung des bei dieser Synthese anfallenden Reaktionsgemisches erfolgt nach üblichen Methoden (vgl. J. Amer. Chem. Soc. 87, 1363 - 1364 (1965)).

Bei der Variante (ß) zur Herstellung der Oxirane der Formel (II) kommt als inertes organisches Lösungsmittel vorzugsweise Acetonitril in Betracht.

Als Base können bei der Verfahrensvariante (ß) starke anorganische oder organische Basen verwendet werden. Vorzugsweise in Frage kommt Natriummethylat.

Die Reaktionstemperaturen können bei der oben beschriebenen Verfahrensvariante (ß) innerhalb eines bestimmten Bereiches variiert werden. Im allgemeinen arbeitet man bei Temperaturen zwischen 0°C und 60°C, vorzugsweise bei Raumtemperatur.

Die Durchführung des Verfahrens zur Herstellung der Oxirane der Formel (II) nach der Variante (ß) und die Aufarbeitung des bei dieser Synthese anfallenden Reaktionsproduktes erfolgt nach üblichen Methoden (vgl. Heterocycles 8, 397 (1977)).

Die Oxirane der Formel (II) können bei dem erfindungsgemäßen Verfahren gegebenenfalls ohne Isolierung direkt weiter umgesetzt werden.

Die außerdem für das erfindungsgemäße Verfahren zur Herstellung der Verbindungen der Formel (I) als Ausgangsstoffe zu verwendenden 1,2,4-Triazole sind durch die Formel (III) allgemein definiert. In dieser Formel steht M vorzugsweise für Wasserstoff, Natrium oder Kalium.

Die 1,2,4-Triazole der Formel (III) sind allgemein bekannte Verbindungen der organischen Chemie.

Als Verdünnungsmittel kommen für das erfindungsgemäße Verfahren unter den Reaktionsbedingungen inerte organische Lösungsmittel in Frage. Hierzu gehören vorzugsweise Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid;

sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens setzt man vorzugsweise auf 1 Mol Oxiran der Formel (II) 1 bis 2 Mol 1,2,4-Triazol und gegebenenfalls katalytische bis 2-molare Mengen an Base ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditions-Salzen der Verbindungen der Formel (I) kommen vorzugsweise folgende Säuren in Frage: Die Halogenwasserstoffsäuren,wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren,wie z.B. die Chlorwasserstoffsäure und die Bromwasserstoffsäure, ferner Phosphorsäure, Salpetersäure und die Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Krankheiten in Getreide und Reiskulturen Verwendung finden; so z.B. gegen Cercosporella-Arten, gegen Cochliobolus sativus und Erysiphe graminis an Gerste und gegen Pyricularia oryzae an Reis. Auch Krankheiten im Gemüseanbau, wie z.B. Gurkenmehltau (Erysiphe cichoracearum) können bekämpft werden.

In bestimmten Anwendungskonzentrationen liegt auch eine wachstumsregulierende Wirkung vor.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1:

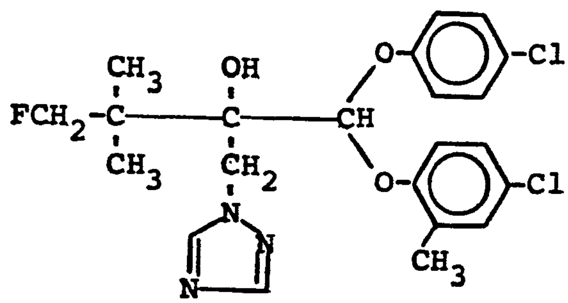

1,0 g (0,043 Mol) Natrium werden in 200 ml n-Butanol gelöst. Zu dieser Lösung fügt man 21 g (0,3 Mol) 1,2,4-Triazol und 40 g (0,1 Mol) 2-(1-Fluor-2-methyl-prop-2-yl)-2-/(4-chlorphenoxy)-(4-chlor-2-methylphenoxy)-methyl/-oxiran hinzu. Nach fünfstündigem Rühren der Mischung unter Rückflußkochen wird das Butanol unter Vakuum weitgehend abgezogen, anschließend wird der Rückstand zwischen Methylenchlorid/Wasser verteilt. Die organische Phase wird sodann mit verdünnter Natronlauge gewaschen, über Magnesiumsulfat getrocknet und anschließend am Rotationsverdampfer vom Lösungsmittel befreit. Der Rückstand wird anschließend über eine kurze Säule mit Kieselgel im System Chloroform/Essigester (4:1) chromatographiert.

Man erhält 19,5 g (das sind 42 % der Theorie) 1-Fluor-2,2-dimethyl-3-hydroxy-3-(1,2,4-triazol-1-yl-methyl)-4-(4-chlorphenoxy)-4-(2-methyl-4-chlorphenoxy)-butan vom Fp. 144°C.

Vorprodukt (V1):

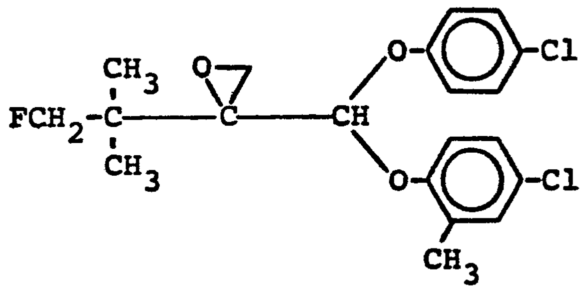

Zu einer Suspension von 13,3 g (0,06 Mol) Trimethylsulfoxoniumiodid in 70 ml Dimethylsulfoxid fügt man unter Rühren bei einer Temperatur von 20°C 2,1 g (0,07 Mol) 80 %iges Natriumhydrid hinzu. Nach 15 Minuten Rühren tropft man dann rasch eine Lösung von 20 g (0,052 Mol) 1-Fluor-2,2-dimethyl-4-(4-chlorphenoxy)-4-(2-methyl-4-chlorphenoxy)-butan-3-on ein und gießt nach einstündigem weiteren Rühren das Reaktionsgemisch in Wasser. Man extrahiert das Rohprodukt mit Methylenchlorid und entfernt das Lösungsmittel am Rotationsverdampfer. Das 2-(1-Fluor-2-methyl-prop-2-yl)-2-/(4-chlorphenoxy)-(4-chlor-2-methylphenoxy)-methyl/-oxiran wird als Öl erhalten, das nach einiger Zeit kristallisiert (Fp. 86°C aus Petrolether). Die Ausbeute beträgt 17,6 g (das sind 85 % der Theorie).

Vorprodukt (V2):

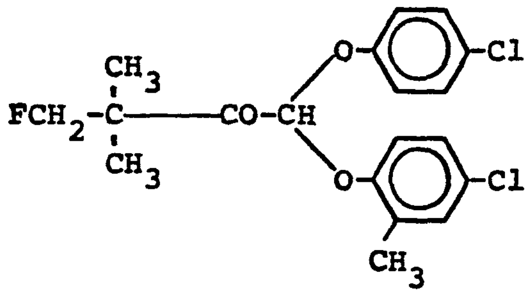

Zu einer gerührten Mischung von 11,4 g (0,08 Mol) 4-Chlor-2-methylphenol und 20 g (0,15 Mol) Kaliumcarbonat in 150 ml Aceton fügt man 20 g (0,06 Mol) 1-Brom-1-(4-chlorphenoxy)-3,3-dimethyl-4-fluor-butan-2-on und kocht 3 Stunden unter Rückfluß. Man gießt in Wasser, extrahiert das Produkt mit Methylenchlorid, wäscht die organische Phase zweimal mit verdünnter Natronlauge und trocknet über Magnesiumsulfat. Nach dem Abziehen des Lösungsmittels im Rotationsverdampfer erhält man 22,3 g (das sind 96 % Rohausbeute) 1-Fluor-2,2-dimethyl-4-(4-chlorphenoxy)-4-(2-methyl-4-chlorphenoxy)-butan-3-on.

Vorprodukt (V3):

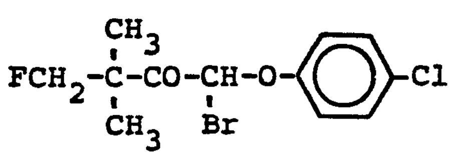

100 g (0,41 Mol) 1-(4-Chlorphenoxy)-3,3-dimethyl-4-fluor-butan-2-on werden in 500 ml Methylenchlorid gelöst bei 20°C unter Rühren mit 21 ml (0,41 Mol) Brom tropfenweise versetzt. Nach vollständiger Entfärbung gießt man in Wasser, trennt die organische Phase ab und wäscht diese mehrmals mit Wasser. Nach dem Trocknen über Magnesiumsulfat wird das Lösungsmittel entfernt und der Rückstand mit Cyclohexan verrührt und abgesaugt. Man erhält 103 g (das sind 78 % der Theorie) 1-Brom-1-(4-chlorphenoxy)-3,3-dimethyl-4-fluor-butan-2-on.

Vorprodukt (V4):

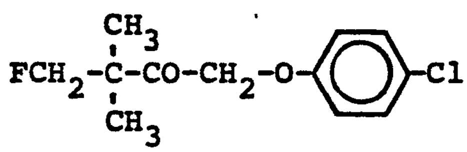

In der Siedehitze tropft man 324 g (1,65 Mol) 1-Brom-3,3-dimethyl-4-fluor-butan-2-on in eine gerührte Mischung von 220 g p-Chlorphenol und 278 g Kaliumcarbonat in 2 l Aceton ein. Nach beendeter Zugabe kocht man noch 10 Stunden unter Rückfluß, saugt die anorganischen Salze ab und engt das Filtrat im Vakuum ein. Den Rückstand verteilt man zwischen Methylenchlorid und Wasser, trennt die organische Phase ab und engt im Vakuum ein. Bei Zugabe von Petrolether kristallisiert das Öl, welches mit Petrolether verrührt und abgesaugt wird. Man erhält 103 g (das sind 78 % der Theorie) 1-(4-Chlorphenoxy)-3,3-dimethyl-4-fluor-butan-2-on vom Fp. 79 - 80°C.

<u>Vorprodukt (V5):</u>

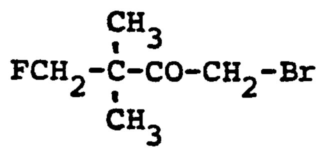

Zu einer Lösung von 236 g (2 Mol) 3,3-Dimethyl-4-fluor-butan-2-on in 500 ml Methylenchlorid tropft man bei 20°C unter Rühren 102 ml (2 Mol) Brom zu. Nach vollständiger Entfärbung wäscht man mit Wasser, trocknet über Magnesiumsulfat und destilliert.

Man erhält 338 g (das sind 87 % der Theorie) 1-Brom-3,3-dimethyl-4-fluor-butan-2-on vom Kp 62 - 66°C/0,3 mm Hg.

Entsprechend Beispiel 1 können die folgenden Verbindungen der allgemeinen Formel

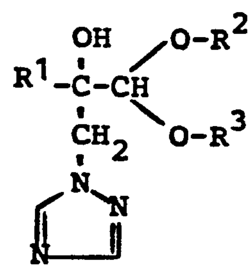

(I)

erhalten werden:

| Bsp. Nr. | $R^1$ | $R^2$ | $R^3$ | Phys. Daten Fp (°C) |
|---|---|---|---|---|
| 2 | $C(CH_3)_3$ | 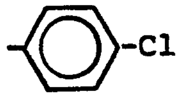 | 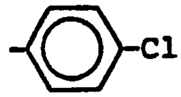 | 113 |
| 3 | $C(CH_3)_3$ | 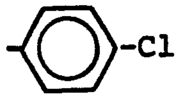 | 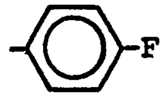 | 100 |
| 4 | $C(CH_3)_3$ | 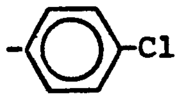 | 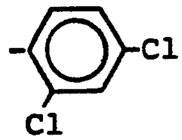 | 125 |
| 5 | $C(CH_3)_3$ | 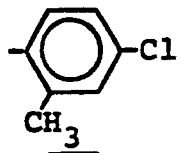 | 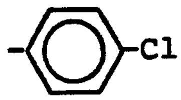 | 145 |
| 6 | $C(CH_3)_3$ | 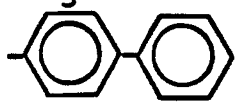 | 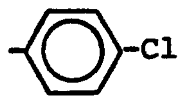 | 88 |
| 7 | $C(CH_3)_3$ | 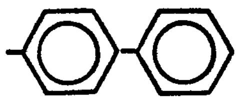 | 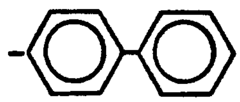 | 162 |
| 8 | $C(CH_3)_3$ | 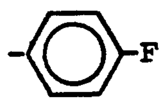 | 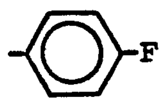 | 85 |
| 9 | $C(CH_3)_3$ | 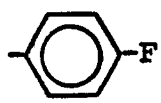 | 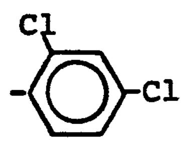 | 110 |
| 10 | $C(CH_3)_3$ | 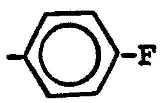 | 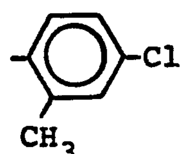 | 106 |
| 11 | $C(CH_3)_3$ | 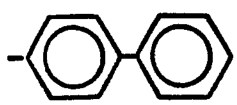 | 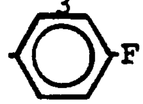 | Harz |

| Bsp. Nr. | $R^1$ | $R^2$ | $R^3$ | Phys. Daten Fp (°C) |
|---|---|---|---|---|
| 12 | $C(CH_3)_3$ | 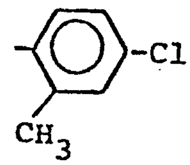 | 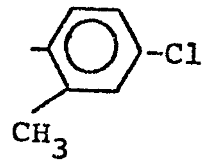 | 145 |
| 13 | $C(CH_3)_3$ | 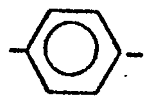 | 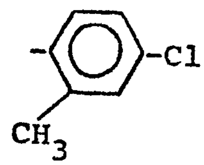 | Harz |
| 14 | $C(CH_3)_3$ | 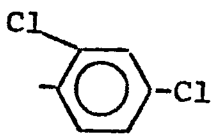 | 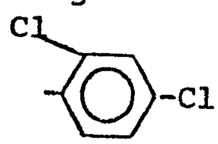 | 128-130 |
| 15 | $C(CH_3)_3$ | 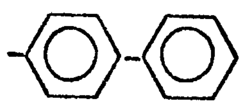 | 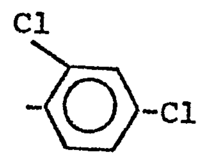 | 154 |
| 16 | $C(CH_3)_3$ | 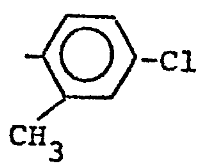 | 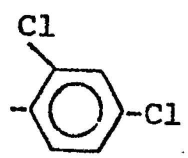 | 138-140 |
| 17 | $(CH_3)_2CH$ | 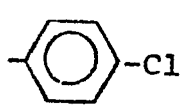 | 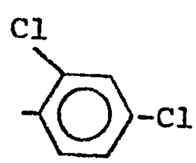 | 134 (Diasteromere Form A) |
| 18 | $(CH_3)_2CH$ | 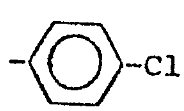 | 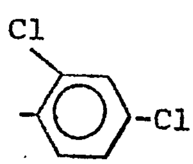 | 112 (Diasteromere Form B) |

| Bsp. Nr. | $R^1$ | $R^2$ | $R^3$ | Phys. Daten Fp (°C) |
|---|---|---|---|---|
| 19 | $(CH_3)_2CH$ | 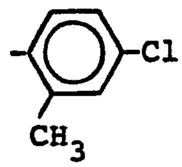 | 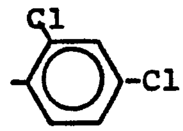 | 118 |
| 20 | $(CH_3)_2CH$ | 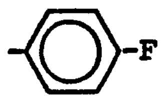 | 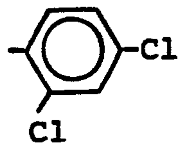 | 82 |
| 21 | $(CH_3)_2CH$ | 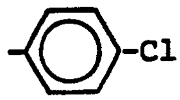 | 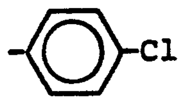 | 116 |
| 22 | 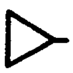 | 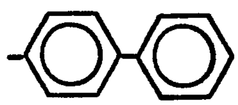 | 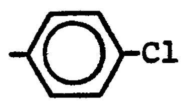 | 111 |
| 23 | $FCH_2-C(CH_3)_2-$ | 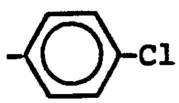 | 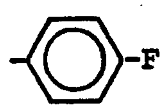 | 82 |
| 24 | $FCH_2-C(CH_3)_2-$ | 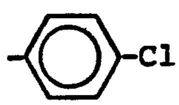 | 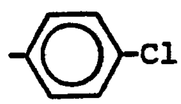 | Harz |
| 25 | $FCH_2-C(CH_3)_2-$ | 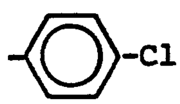 | 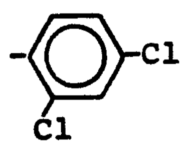 | 119 |
| 26 | $FCH_2-C(CH_3)_2-$ | 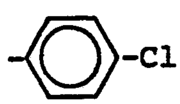 | 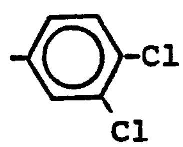 | 122 |
| 27 | $FCH_2-C(CH_3)_2-$ | 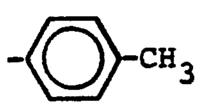 | 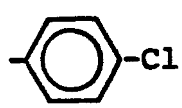 | 80 |

| Bsp. Nr. | $R^1$ | $R^2$ | $R^3$ | Phys. Daten Fp (°C) |
|---|---|---|---|---|
| 28 | $FCH_2-C(CH_3)_2-$ | 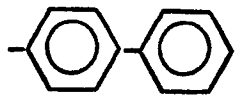 | 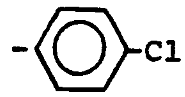 | Harz |
| 29 | 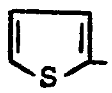 | 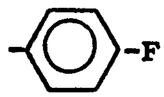 | 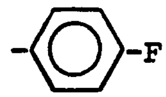 | 85 |
| 30 | 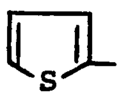 | 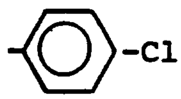 | 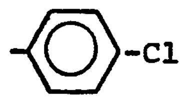 | 106 |
| 31 | 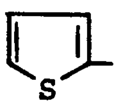 | 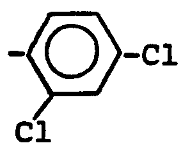 | 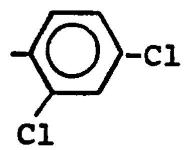 | 188 |
| 32 | 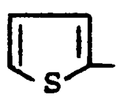 | 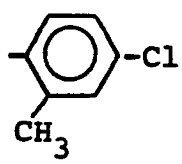 | 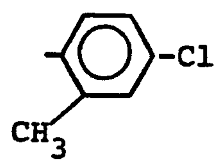 | 160 |
| 33 | $FCH_2-C(CH_3)_2-$ | 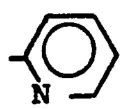 | 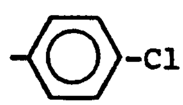 | 130 |
| 34 | 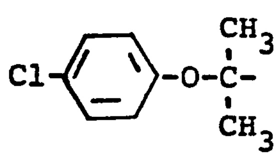 | 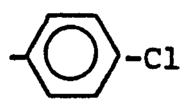 | 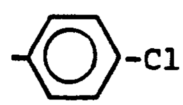 | 103 |
| 35 | 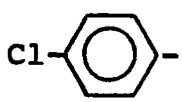 | 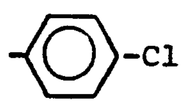 | 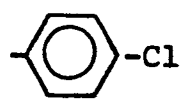 | 57-63 |

Verwendungsbeispiele

In den nachfolgenden Beispielen werden folgende Verbindungen als Vergleichsverbindungen eingesetzt:

(A) 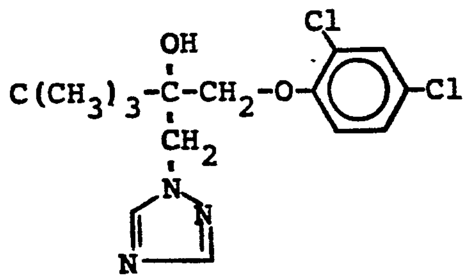

Beispiel A

Cochliobolus sativus-Test (Gerste)/protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 15, 16, 22, 23, 24, 27, 29, 30, 31, 32.

Tabelle A

Cochliobolus sativus-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 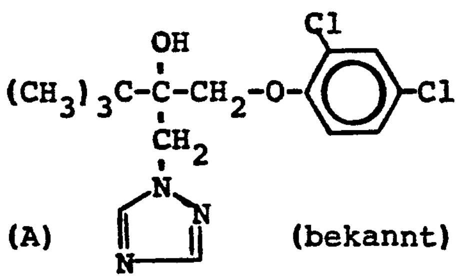 (A) (bekannt) | 0,025 | 100 |
| 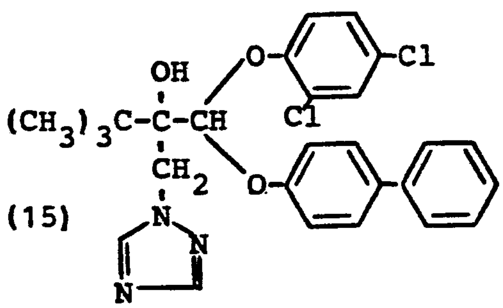 (15) | 0,025 | 50 |
| 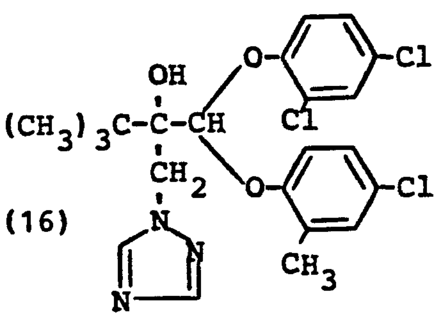 (16) | 0,025 | 50 |

Tabelle A (Fortsetzung)

Cochliobolus sativus-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 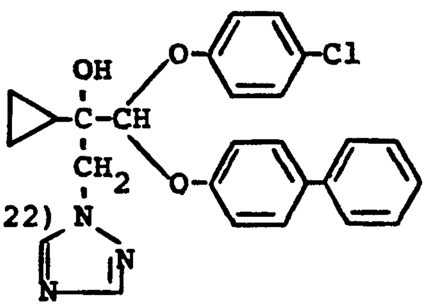 (22) | 0,025 | 33,7 |
| 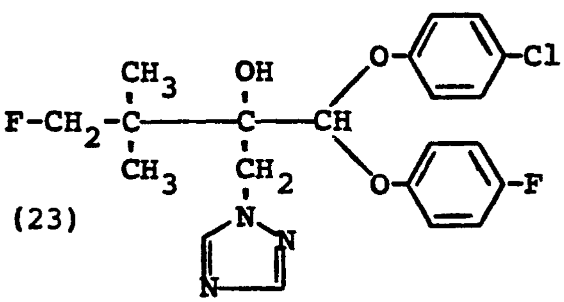 (23) | 0,025 | 50 |
| 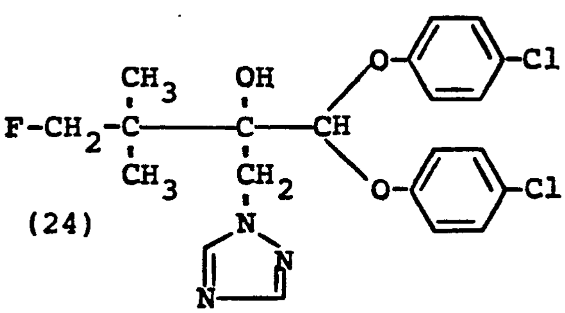 (24) | 0,025 | 50 |

Tabelle A (Fortsetzung)

Cochliobolus sativus-Test (Gerste) / protektiv

| Wirkstoff | Wirkstoff-konzentration in der Spritzbrühe in Gew.-% | Krankheits-befall in % der unbehan-delten Kon-trolle |
|---|---|---|
| 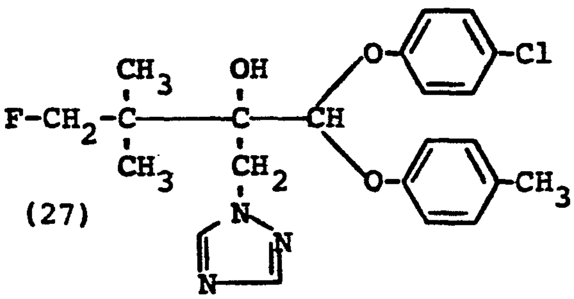 (27) | 0,025 | 41,2 |
| 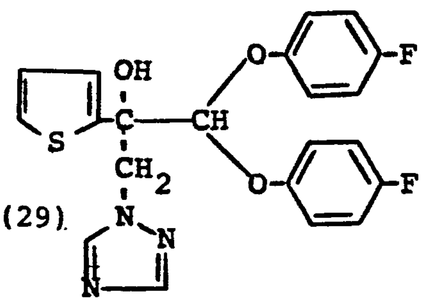 (29) | 0,025 | 41,2 |
| 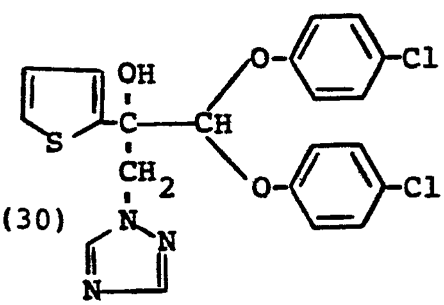 (30) | 0,025 | 50 |

Tabelle A (Fortsetzung)

Cochliobolus sativus-Test (Gerste) / protektiv

| Wirkstoff | Wirkstoff-konzentration in der Spritzbrühe in Gew.-% | Krankheits-befall in % der unbehan-delten Kon-trolle |
|---|---|---|
| 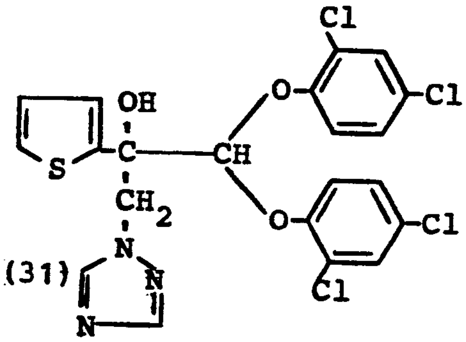 (31) | 0,025 | 25 |
| 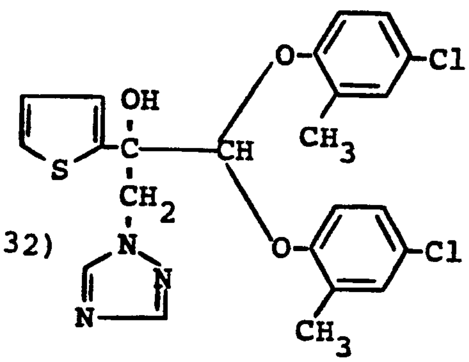 (32) | 0,025 | 25 |

Patentansprüche

1) Triazolylmethylcarbinol-arylacetale der allgemeinen Formel (I)

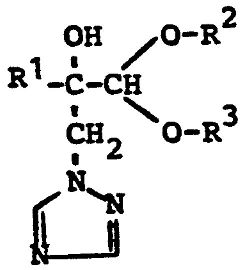

(I)

in welcher

$R^1$ für jeweils gegebenenfalls substituiertes Alkyl, Alkenyl, Cycloalkyl, Heterocyclyl oder Aryl steht und

$R^2$ und $R^3$ unabhängig voneinander für jeweils gegebenenfalls substituiertes Aryl oder Heterocyclyl stehen,

und deren Säureadditions-Salze und Metallsalz-Komplexe.

2) Verbindungen der allgemeinen Formel (I) in Anspruch 1,

wobei

$R^1$ für geradkettiges oder verzweigtes Alkyl mit 1 bis 4 Kohlenstoffatomen und für gegebenenfalls einfach oder mehrfach, gleich oder verschieden substituiertes Cycloalkyl mit 3 bis 7 Kohlenstoffatomen steht, wobei als Substituenten genannt seien: Halogen, Alkyl mit 1 bis 4 Kohlenstoffatomen und Alkoxy mit 1 oder 2 Kohlenstoffatomen, ferner für gegebenenfalls einfach oder mehrfach, gleich oder verschieden substituiertes Phenyl bzw. fünfgliedriges Heterocyclyl mit einem Sauerstoff- oder Schwefelatom oder ein oder zwei Stickstoffatomen steht, wobei als jeweilige Substituenten die unten bei $R^2$ genannten Phenylsubstituenten in Frage kommen, sowie für die Gruppierungen

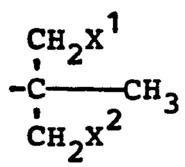 und 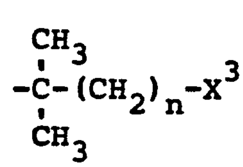 steht,

wobei

$X^1$ für Halogen steht,

$X^2$ für Wasserstoff oder Halogen steht,

$X^3$ für Alkyl, Alkoxy und Alkylthio mit jeweils 1 bis 4 Kohlenstoffatomen, für Halogenalkoxy und Halogenalkylthio mit jeweils 1 oder 2 Kohlenstoffatomen und 1 bis 5 gleichen oder verschiedenen Halogenatomen, ferner für Alkenyl mit 2 bis

6 Kohlenstoffatomen, für Alkoxycarbonyl mit 1 bis 4 Kohlenstoffatomen im Alkylteil, für Cyano sowie für gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten die unten bei $R^2$ genannten Phenylsubstituenten in Frage kommen, und

n für die Zahlen 0, 1 oder 2 steht,

$R^2$ und $R^3$ für Phenyl oder für sechsgliedriges Heterocyclyl mit einem oder zwei Stickstoffatomen steht, wobei diese Reste jeweils einfach oder mehrfach substituiert sein können durch Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, durch Cyclohexyl, Dialkylamino mit 1 bis 4 Kohlenstoffatomen in jedem Alkylteil, durch Nitro, Cyano, Alkoxycarbonyl mit 1 bis 4 Kohlenstoffatomen im Alkylteil, sowie durch gegebenenfalls durch Halogen substituiertes Phenyl.

3) Verfahren zur Herstellung von Triazolylmethylcarbinol-arylacetalen der Formel (I)

0182188



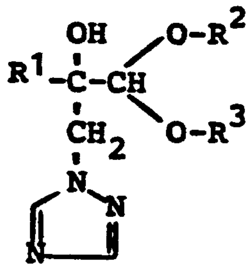

(I)

in welcher

$R^1$ für jeweils gegebenenfalls substituiertes Alkyl, Alkenyl, Cycloalkyl, Heterocyclyl oder Aryl steht, und

$R^2$ und $R^3$ unabhängig voneinander für jeweils gegebenenfalls substituiertes Aryl oder Heterocyclyl stehen,

sowie deren Säureadditions-Salzen und Metallsalzkomplexen,

dadurch gekennzeichnet, daß man Oxirane der Formrl (II)

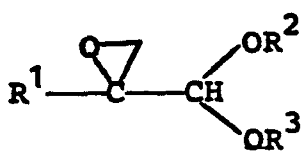

(II)

in welcher

$R^1$, $R^2$ und $R^3$ die oben angegebene Bedeutung haben,

mit 1,2,4-Triazol der Formel (III)

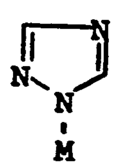

(III)

in welcher

M für Wasserstoff oder ein Alkalimetalläquivalent steht,

in Gegenwart eines Verdünnungsmittels und gegebenenfalls in Gegenwart einer Base umsetzt, und gegebenenfalls noch anschließend eine Säure oder ein Metallsalz addiert.

4) Fungizide Mittel, gekennzeichnet durch einen Gehalt an mindestens einem Triazolylmethylcarbinolarylacetal der Formel (I) bzw. einem Säureadditions-Salz oder Metallsalz-Komplex eines Triazolylmethylcarbinol-arylacetals der Formel (I).

5) Verfahren zur Bekämpfung von Pilzen, dadurch gekennzeichnet, daß man Triazolylmethylcarbinolarylacetale der Formel (I) bzw. deren Säureadditions-Salze oder Metallsalz-Komplexe auf Pilze oder ihren Lebensraum einwirken läßt.

6) Verwendung von Triazolylmethylcarbinol-arylacetalen der Formel (I) bzw. von deren Säureadditions-Salzen oder Metallsalz-Komplexen als Pflanzenschutzmittel.

7) Verwendung von Triazolylmethylcarbinol-arylacetalen der Formel (I) bzw. von deren Säureadditions-Salzen oder Metallsalz-Komplexen zur Bekämpfung von Pilzen.

8) Verfahren zur Herstellung von fungiziden Mitteln, dadurch gekennzeichnet, daß man Triazolylmethylcarbinol-arylacetale der Formel (I) bzw. deren Säureadditions-Salze oder Metallsalz-Komplexe mit Streckmitteln und/oder oberflächenaktiven Mitteln vermischt.